# EUROPEAN PATENT APPLICATION

(11) **EP 4 353 273 A1**
(43) Date of publication of application: **17.04.2024**
(21) Application number: 22820000.2
(22) Date of filing: 13.05.2022
(51) Int. Cl.: A61L 101/22, B65B 55/10, A61L 2/20

(54) **DISINFECTANT VAPORIZING DEVICE AND DISINFECTANT VAPORIZING METHOD**

(30) Priority: 11.06.2021 JP 2021097885
(71) Applicant: DAI NIPPON PRINTING CO., LTD., Tokyo 162-8001 (JP)
(72) Inventor: HAYAKAWA Atsushi, Tokyo 162-8001 (JP); SATO Yoshinori, Tokyo 162-8001 (JP); KAWAI Yasuhiro, Tokyo 162-8001 (JP)
(74) Representative: Müller-Boré & Partner Patentanwälte PartG mbB
(86) International application number: PCT/JP2022/020219
(87) International publication number: WO 2022/259817

(57) **Abstract**

To provide a sterilizer gasification apparatus that gasifies a sterilizer with reliability, improves the concentration of the sterilizer in the gas and is compact and a sterilizer gasification method. A helical metal pipe that meets at least one of conditions that a pitch of the helical pipe is equal to or greater than 1.0 mm and equal to or smaller than 30.0 mm and that an angle of the helix is greater than 0 degrees and equal to or smaller than 30 degrees with respect to a plane perpendicular to a central axis of the helix, a solidified molten metal layer in which the helical metal pipe is embedded in a casting process, a heating body that heats an outer surface of the solidified molten metal layer, and a nozzle that is at one end of the helical metal pipe and sprays a sterilizer into the helical metal pipe are provided.

## Description

### Technical Field

The present disclosure relates to a sterilizer gasification apparatus and a sterilizer gasification method for producing gas of a sterilizer used for sterilization of a packaged material in an aseptic filling and packaging machine.

### Background Art

Foods and drinks packed in various containers by aseptic filling and packaging machines, such as milk portions, carton drinks, pouched soups, cup drinks, and drinks in PET or other plastic bottles, are put in general circulation. The aseptic filling and packaging machine is an apparatus that fills a container sterilized in an aseptic atmosphere with a sterilized content and seals the container. Products produced by the aseptic filling and packaging machine can be distributed and stored at room temperature and therefore require less energy consumption than refrigerated or frozen products. Such products also taste good and thus are increasing.

The aseptic filling and packaging machine uses a variety of packaging materials as containers as described above, and different sterilization methods are used for different packaging materials. Although there are methods in which ultraviolet rays or electron beams are applied, a mainstream method is to sterilize the surface of the packaging material with a sterilizer. In sterilization of a packaging material with a sterilizer, containers of milk portions or carton drinks are immersed in a sterilizer for sterilization, although a sterilizer may be alternatively sprayed to containers. Flat packaging materials that can resist a relatively high drying temperature after the immersion are immersed in a sterilizer for sterilization. On the other hand, shaped containers, such as cups or bottles, or packaging material, such as a film, that expand in high-temperature drying are sterilized by spraying of a sterilizer.

If the droplets of the sprayed sterilizer are large, the droplets may run on the side surface of the cup or bottle. The finer the droplets of the sprayed sterilizer, the more uniformly the sterilizer is applied to the surface of the packaging material, and the higher the sterilization effect is. In view of this, a method has been proposed that makes the droplets of the sterilizer finer (Patent Literature 1).

The finer the droplets of the sterilizer on the surface of the packaging material, and the more densely the surface of the packaging material is covered with the droplets of the sterilizer, the higher the sterilization effect is. In view of this, a method has been proposed in which rather than spraying droplets of the sterilizer, the sterilizer is gasified, and the sterilizer gas is blown on the surface of the packaging material and the sterilizer is condensed on the surface of the packaging material (Patent Literature 2). The gasification of the sterilizer is achieved by dripping the sterilizer onto a heated heat generating body.

Furthermore, a method has been proposed in which a large amount of sterilizer is efficiently gasified by spraying the sterilizer into a heated pipe (Patent Literature 3). Furthermore, a method has been proposed in which a heat storage body is provided in a heated pipe (Patent Literature 4).

The sterilizer vaporization apparatus described in Patent Literature 3 sprays a sterilizer into a heated cylinder. Furthermore, a sterilizer gasification apparatus has also been proposed in which a sterilizer is sprayed into a heated cylinder, hot air is blown from the upstream side, and a plate heater is provided on a lower part of the cylinder (Patent Literature 5). An apparatus also has been proposed that vaporizes and discharges a hydrogen peroxide solution by injecting the hydrogen peroxide solution onto a surface of a heating apparatus while supplying a gas medium into a cylinder (Patent Literature 6).

The sterilizer gasification apparatus described above gasifies a sterilizer by bringing the sterilizer into contact with a surface of a heated relatively large cylindrical tube or a heated housing. In this process, hot air may be blown. Furthermore, an apparatus has been proposed that gasifies a sterilizer by blasting or spraying the sterilizer into a heated relatively narrow flow channel. According to Patent Literature 7, a coil spring is inserted between an outer tube heated and a revolving body inside, and a sterilizer is vaporized by being flowed in the helical space formed by the coil spring. Patent Literature 8 proposes an apparatus in which a sterilizer is sprayed into a circumferential groove and flows in a plurality of heated pipes extending from the bottom of the groove.

Furthermore, in a vaporizer for vaporizing a sterilized gas from a raw gas, a method is described in which a pipe-like member forming a helical vaporizing chamber is made of an inexpensive material having high thermal conductivity and is embedded in the vaporizer in a casting process (Patent Literature 9). Furthermore, a sterilizer gasification apparatus has been proposed that includes a solidified molten metal layer that covers a helical metal pipe, a heating body that heats the outer surface of the solidified molten metal layer, and a nozzle that is provided at one end of the helical metal pipe and sprays a sterilizer into the helical metal pipe (Patent Literature 10).

A hydrogen peroxide solution is used as the sterilizer, and hydrogen peroxide is decomposed by trace amounts of heavy metals contained in the hydrogen peroxide solution. To prevent the decomposition, sodium pyrophosphate or orthophosphoric acid, which has been proven to be safe and effective as a stabilizer, is added to the hydrogen peroxide solution used for sterilization in the aseptic filling and packaging machine (Patent Literature 11). When the hydrogen peroxide solution is gasified, such a stabilizer may be deposited and accumulated on the surface of the heating body that causes the gasification, thereby decreasing the efficiency of the gasification of the hydrogen peroxide solution or causing a blockage of the nozzle for blowing the gas of the hydrogen peroxide solution to the object to be sterilized. To prevent the adverse effects of the deposition of the stabilizer, a method has been proposed in which the hydrogen peroxide solution is once gasified and then cooled and passed through a filter, and the hydrogen peroxide solution liquefied again is gasified again (Patent Literature 12).

### Citation List

### Patent Literature

Patent Literature 1: Japanese Patent Laid-Open No. S60-220067
Patent Literature 2: Japanese Patent Laid-Open No. S63-11163
Patent Literature 3: Japanese Patent Laid-Open No. H03-224469
Patent Literature 4: Japanese Patent Laid-Open No. H10-218134
Patent Literature 5: Japanese Patent Laid-Open No. 2001-276189
Patent Literature 6: Japanese Translation of PCT International Application Publication No. 2010-534167
Patent Literature 7: Japanese Patent Laid-Open No. S62-122926
Patent Literature 8: Japanese Translation of PCT International Application Publication No. 2005-503206
Patent Literature 9: Microfilm of Japanese Utility Model Application No. S53-17908
Patent Literature 10: Japanese Patent Laid-Open No. 2021-029541
Patent Literature 11: Japanese Patent Laid-Open No. 2006-240969
Patent Literature 12: Japanese Patent Laid-Open No. H10-258811

### Summary of Invention

### Technical Problem

Many aseptic filling and packaging machines use a gasified hydrogen peroxide solution for sterilization of packaging materials. The hydrogen peroxide solution is gasified by bringing the hydrogen peroxide solution into contact with a surface of a heated heat generating body. Although hot air may be blown on a sprayed hydrogen peroxide solution, the hot air has low heat capacity, and therefore it is difficult to completely gasify the hydrogen peroxide solution. The hydrogen peroxide solution sprayed into a cylindrical tube comes into contact with the inner surface of the heated cylindrical tube and is gasified. Some of the sprayed hydrogen peroxide solution does not come into contact with the inner surface of the cylindrical tube and is blown on the container to be sterilized in the form of droplets.

The sterilization effect is improved by the sprayed hydrogen peroxide solution being completely gasified and coming into contact with bacteria or the like on the surface of a container or a flat packaging material yet to be shaped into a container, and is also improved by the gasified hydrogen peroxide solution being condensed on the surface of the container to form fine droplets and the droplets of hydrogen peroxide solution being gasified again. That is, the sterilization effect is improved by completely gasifying a hydrogen peroxide solution and thereby increasing the concentration of hydrogen peroxide in the resulting gas.

To increase the gasification efficiency of the hydrogen peroxide solution, it is advantageous to increase the surface area of the cylindrical tube with respect to the volume of the sprayed hydrogen peroxide solution so that all droplets of the hydrogen peroxide solution come into contact with the surface of the heated heat generating body. However, if the length of the cylindrical tube is increased, the hydrogen peroxide solution once gasified may be condensed again in the space in the cylindrical tube, and it is difficult to completely gasify the hydrogen peroxide solution. If the diameter of the cylindrical tube is increased, some of the sprayed hydrogen peroxide solution may fail to reach the inner surface of the cylindrical tube. Increasing the length or diameter of the cylindrical tube leads to an increased size of the gasification apparatus and therefore is not preferable for the aseptic filling machine.

On the other hand, the gasification apparatuses proposed in Patent Literatures 7 and 8 gasify a hydrogen peroxide solution by passing the hydrogen peroxide solution through a helical groove or conduit. Compared with the gasification apparatus formed by a cylindrical tube, the surface area of the heated heating body with respect to the volume of the sprayed hydrogen peroxide solution is large, so that the gasification efficiency of the hydrogen peroxide solution is estimated to be high. However, both the apparatuses have a complicated structure and require a high manufacturing cost.

The sterilizer gasification apparatuses described in Patent Literatures 9 and 10, in which a helical pipe is embedded in a metal, and the metal with the helical pipe embedded therein is heated to vaporize a sterilizer in the helical pipe, can efficiently vaporize the sterilizer. However, how to increase the efficiency of the sterilizer gasification apparatus and how to reduce the size of the sterilizer gasification apparatus are not disclosed.

The present disclosure has been devised to solve the problems described above, and an object of the present disclosure is to provide a sterilizer gasification apparatus that can gasify a sterilizer with reliability and increase the concentration of the sterilizer in the resulting gas by increasing the chance for small droplets of the sprayed sterilizer to come into contact with a heated heat generating body, has high maintainability and is compact, and a sterilizer gasification method.

When a hydrogen peroxide solution is used as the sterilizer, the stabilizer contained in the hydrogen peroxide solution is deposited on the inner surface of the gasification apparatus and accumulated on the surface of the heated heat generating body. The accumulated stabilizer inhibits the heat transfer to the outermost surface of the heated heat generating body and decreases the gasification efficiency of the hydrogen peroxide solution. To prevent this, the sterilizer gasification apparatus has to be regularly disassembled and cleaned, which reduces the productivity. According to the present disclosure, the manufacturing cost is reduced, so that the gasification part of the gasification apparatus in which the sterilizer is deposited can be replaced after a certain period of use, rather than being disassembled and cleaned.

### Solution to Problem

A sterilizer gasification apparatus according to an embodiment includes a helical metal pipe that meets at least one of conditions that a pitch of a helical pipe of the helical metal pipe is equal to or greater than 1.0 mm and equal to or smaller than 30.0 mm and that an angle of a helix of the helical metal pipe is greater than 0 degrees and equal to or smaller than 30 degrees with respect to a plane perpendicular to a central axis of the helix, a solidified molten metal layer in which a helical part of the helical metal pipe is embedded in a casting process, two end parts of the helical metal pipe not being embedded in the solidified molten metal layer, a heating body that heats an outer surface of the solidified molten metal layer, and a nozzle that is provided at one end of the helical metal pipe and sprays a sterilizer into the helical metal pipe.

In the sterilizer gasification apparatus according to the embodiment, preferably, the helical metal pipe is made of stainless steel.

In the sterilizer gasification apparatus according to the present disclosure, preferably, a sterilizer gas ejection port, from which a sterilizer gas is ejected, at another end of the helical metal pipe is provided with a gasification apparatus tip end nozzle that guides the ejected sterilizer gas.

In the sterilizer gasification apparatus according to the embodiment, preferably, the gasification apparatus tip end nozzle is made of stainless steel or aluminum.

In the sterilizer gasification apparatus according to the embodiment, preferably, a direction of the helix of the helical metal pipe changes at least once.

In the sterilizer gasification apparatus according to the embodiment, preferably, the solidified molten metal layer is made of brass, aluminum or an aluminum alloy.

In the sterilizer gasification apparatus according to the embodiment, preferably, the solidified molten metal layer and the heating body that heats the outer surface of the solidified molten metal layer are made of a same material.

In the sterilizer gasification apparatus according to the embodiment, preferably, the solidified molten metal layer has the shape of a circular column and covers an outer periphery and an inside of the helical metal pipe.

In the sterilizer gasification apparatus according to the embodiment, preferably, the solidified molten metal layer has the shape of a circular tube and covers an outer periphery and an inner periphery of the helical metal pipe.

In the sterilizer gasification apparatus according to the embodiment, preferably, the nozzle is a two-fluid spray.

In the sterilizer gasification apparatus according to the embodiment, preferably, a cleaning liquid pouring apparatus that pours a cleaning liquid into the helical metal pipe is provided.

In the sterilizer gasification apparatus according to the embodiment, preferably, a sterilizer gas ejection port from which a gas of the sterilizer gasified in the helical metal pipe is ejected is provided with a branch pipe, on which one or more of a hydrogen peroxide gas concentration meter, a thermometer and a conductivity meter is provided.

In a sterilizer gasification method according to an embodiment, a helical metal pipe that meets a condition that a pitch of a helical pipe of the helical metal pipe is equal to or greater than 1.0 mm and equal to or smaller than 30.0 mm or that an angle of a helix of the helical metal pipe is greater than 0 degrees and equal to or smaller than 30 degrees with respect to a plane perpendicular to a central axis of the helix, a solidified molten metal layer in which a helical part of the helical metal pipe is embedded in a casting process, two end parts of the helical metal pipe not being embedded in the solidified molten metal layer, and a heating body that heats an outer surface of the solidified molten metal layer are provided, and a sterilizer is sprayed into the helical metal pipe from one end of the helical metal pipe.

In the sterilizer gasification method according to the embodiment, preferably, the helical metal pipe is made of stainless steel.

In the sterilizer gasification method according to the embodiment, preferably, a sterilizer gas ejection port, from which a sterilizer gas is ejected, at another end of the helical metal pipe is provided with a gasification apparatus tip end nozzle to guide the ejected sterilizer gas.

In the sterilizer gasification method according to the embodiment, preferably, the gasification apparatus tip end nozzle is made of stainless steel or aluminum.

In the sterilizer gasification method according to the embodiment, preferably, a direction of the helix of the helical metal pipe changes at least once.

In the sterilizer gasification method according to the embodiment, preferably, the solidified molten metal layer is made of brass, aluminum or an aluminum alloy.

In the sterilizer gasification method according to the embodiment, preferably, the solidified molten metal layer and the heating body that heats the outer surface of the solidified molten metal layer are made of a same material.

In the sterilizer gasification method according to the embodiment, preferably, the solidified molten metal layer has the shape of a circular column and covers an outer periphery and an inside of the helical metal pipe.

In the sterilizer gasification method according to the embodiment, preferably, the solidified molten metal layer has the shape of a circular tube and covers an outer periphery and an inner periphery of the helical metal pipe.

In the sterilizer gasification method according to the embodiment, preferably, the sterilizer is sprayed into the helical metal pipe by a two-fluid spray.

In the sterilizer gasification method according to the embodiment, preferably, a cleaning liquid is poured into the helical metal pipe.

In the sterilizer gasification method according to the embodiment, preferably, water used for the cleaning liquid is ion-exchanged water, reverse osmosis-filtered water or distilled water.

In the sterilizer gasification method according to the embodiment, preferably, after the cleaning liquid is poured into the helical metal pipe, the cleaning liquid is rinsed away with water, and air is blown into the helical metal pipe to remove remaining water.

### Advantageous Effects of Invention

The sterilizer gasification apparatus according to the present disclosure includes a helical metal pipe, a solidified molten metal layer in which the helical metal pipe is embedded in a casting process, a heating body that heats the outer surface of the solidified molten metal layer, and a nozzle that is provided at one end of the helical metal pipe and sprays a sterilizer into the helical metal pipe, and can gasify the sterilizer sprayed into the helical metal pipe with reliability. Therefore, the concentration of the sterilizer in the gas can be increased, and the sterilization effect can be improved. Furthermore, since the sterilizer is gasified with reliability, the gasified sterilizer is condensed on the surface of the container to form fine droplets, and the droplets of the sterilizer are gasified again by the subsequent heating, so that the sterilization effect is improved.

By reducing the pitch between the turns of the helical metal pipe and/or reducing the angle of the helix, the helical metal pipe can be made compact even if the metal pipe has a sufficient length, and a sterilizer gasification apparatus that is smaller but can gasify a larger amount of sterilizer than conventional sterilizer gasification apparatuses can be provided. Since the helical metal pipe is compact, the ease of replacement of the sterilizer gasification apparatus can be improved, and the structure for holding the sterilizer gasification apparatus can be simplified.

In addition, the sterilizer gasification apparatus according to the present disclosure requires low manufacturing cost. Therefore, when a hydrogen peroxide solution is used as the sterilizer, the gasification part of the gasification apparatus in which the stabilizer contained in the hydrogen peroxide solution is deposited does not have to be disassembled and cleaned when it seems that the stabilizer is deposited in the helical metal pipe, and can be replaced after a certain period of use. Therefore, the productivity of the aseptic filling and packaging machine can be improved.

In addition, the sterilizer gasification apparatus according to the present disclosure has the helical metal pipe as an internal structure. Therefore, compared with the gasification apparatus having a cylindrical shape, the sterilizer gasification apparatus according to the present disclosure is superior in cleaning effect with a cleaning liquid and in ease of rinsing, and the stabilizer can be efficiently removed in a short time, and the chemicals can be efficiently rinsed away in a short time.

### Brief Description of Drawings

[FIG. 1] FIG. 1 is a front view of a sterilizer gasification apparatus according to an embodiment 1.
[FIG. 2] FIG. 2 is a plan view of the sterilizer gasification apparatus according to the embodiment 1.
[FIG. 3] FIG. 3 is a perspective view of a helical metal pipe of the sterilizer gasification apparatus according to the embodiment 1.
[FIG. 4] FIG. 4 is a perspective view of another helical metal pipe of the sterilizer gasification apparatus according to the embodiment 1.
[FIG. 5] FIG. 5 is a front view of a sterilizer gasification apparatus according to an embodiment 2.
[FIG. 6] FIG. 6 is a plan view of the sterilizer gasification apparatus according to the embodiment 2.
[FIG. 7] FIG. 7 is front view of the sterilizer gasification apparatus according to the embodiment 1 provided with a cleaning liquid pouring apparatus.

### Description of Embodiments

In the following, embodiments of the present disclosure will be described with reference to the drawings.

### (Embodiment 1)

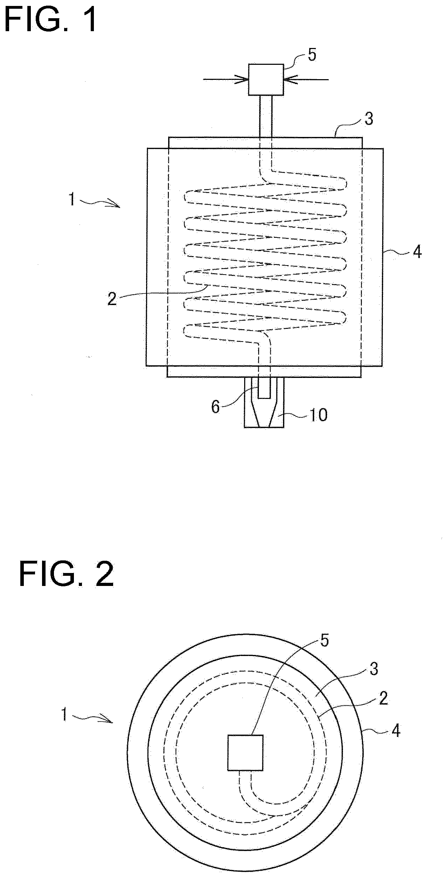

FIG. 1 shows a sterilizer gasification apparatus 1 according to an embodiment 1. The sterilizer gasification apparatus 1 includes a helical metal pipe 2, a solidified molten metal layer 3 in which the helical metal pipe 2 is embedded in a casting process, a heating body 4 that heats an outer surface of the solidified molten metal layer 3, and a nozzle 5 that sprays a sterilizer into the helical metal pipe 2 provided at one end of the helical metal pipe 2.

An outer periphery of the helical metal pipe 2 is heated by heat transferred from the heating body 4 provided on the outer surface of the solidified molten metal layer 3 in which the helical metal pipe 2 is embedded in a casting process. A sterilizer is sprayed into the helical metal pipe 2 from the nozzle 5 provided at one end of the helical metal pipe 2, and the sterilizer can be gasified. The sprayed sterilizer comes into contact with an inner surface of the heated helical metal pipe 2 and is thereby vaporized and gasified.

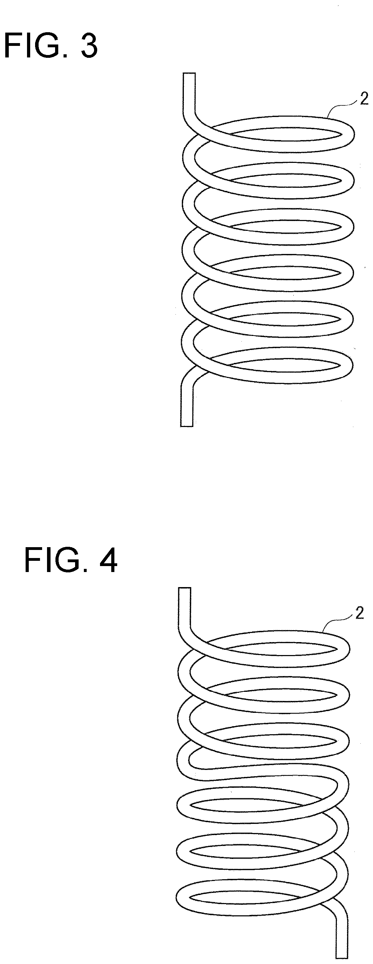

As shown in FIG. 3, the helical metal pipe 2 is a metal pipe worked into a helical shape. The helical metal pipe can be made of any metal having a melting point higher than the melting point of the material forming the solidified molten metal layer 3. For example, the metal may be iron, stainless steel, copper, brass, or titanium, and the stainless steel is preferable since the stainless steel has high durability against sterilizers and cleaning liquids containing chemicals. The stainless steel may be an alloy of iron, chromium and nickel, an alloy of iron, chromium, nickel and molybdenum, an alloy of iron, chromium, nickel, molybdenum and copper, an alloy of iron, chromium, nickel, molybdenum and nitrogen, or a duplex stainless steel alloy having two structures, that is, any of the stainless steel alloys described above and ferrite.

The helical metal pipe 2 is worked into a helical shape in order to ensure as long a flow channel as possible for the sterilizer in the limited space for spraying of the sterilizer into the metal pipe and gasification of the sprayed sterilizer. The helical metal pipe 2 is heated from outside and the sprayed sterilizer passes through the heated helical metal pipe 2, so that the sterilizer is gasified with reliability.

The helical shape of the helical metal pipe 2 meets at least one of conditions that the pitch of the helical pipe of the helical metal pipe 2 is equal to or greater than 1.0 mm and equal to or smaller than 30.0 mm and that the angle of the helix of the helical metal pipe 2 with respect to a plane perpendicular to the central axis of the helix is greater than 0 degrees and equal to or smaller than 30 degrees. By reducing the pitch of the helical pipe and/or reducing the angle of the helix, the helical metal pipe 2 can be made more compact for the same length. By making the helical metal pipe 2 more compact, the sterilizer gasification apparatus 1 can also be made more compact for the same amount of sterilizer to be gasified, the solidified molten metal layer 3 in which the helical metal pipe 2 is embedded can be made smaller, and the weight of the sterilizer gasification apparatus 1 can be reduced.

The pitch of the helical pipe is equal to or greater than 1.0 mm and equal to or smaller than 30.0 mm. The pitch of the helical pipe means the shortest distance between adjacent turns of the pipe at an arbitrary point. If the pitch of the helical pipe is smaller than 1.0 mm, there is a possibility that the distance of the solidified molten metal layer 3 between the turns of the pipe is small and the strength of the solidified molten metal layer 3 decreases. In addition, the helical metal pipe 2 serves as a heat insulating layer and inhibits the high temperature of the heated outer peripheral surface of the solidified molten metal layer 3 from being transferred to the interior (the central part). If the pitch of the helical pipe is greater than 30.0 mm, the dimension of the helical metal pipe 2 in the axial direction is too large, and the size of the sterilizer gasification apparatus 1 is also too large.

The pitch of the helical pipe does not have to be fixed but can vary between the two ends of the helical metal pipe 2. When the pitch of the helical pipe varies, the sterilizer flows irregularly, the sterilizer coming into contact with the wall surface of the helical metal pipe 2 is stirred, and the vaporization efficiency of the sterilizer is improved.

The angle of the helix is greater than 0 degrees and equal to or smaller than 30 degrees with respect to the plane perpendicular to the central axis of the helix. Preferably, the angle is equal to or greater than 5 degrees and equal to or smaller than 30 degrees. If the angle of the helix is smaller than 5 degrees, there is a possibility that the distance between adjacent turns of the pipe cannot be maintained. In addition, the sterilizer sprayed into the pipe and the sterilizer vaporized in the pipe does not smoothly flow. Thus, the pressure of the compressed air used for the spraying needs to be increased, and the ejection pressure of the sterilizer gas ejected from a sterilizer gas ejection port 6 increases, so that the amount of the sterilizer gas is difficult to adjust. If the angle is greater than 30 degrees, the dimension of the helical metal pipe 2 in the axial direction is too large.

The angle of the helix does not have to be fixed but can vary between the two ends of the helical metal pipe 2. When the angle of the helix varies, the sterilizer flows irregularly, the sterilizer coming into contact with the wall surface of the helical metal pipe 2 is stirred, and the vaporization efficiency of the sterilizer is improved.

The angle of the first turn of the helix from each of the two ends of the helical metal pipe 2 can be greater than 0 degrees and smaller than 5 degrees. The first turn from the end of the helix does not interfere with the adjacent pipe even if the angle of the helix is small. By setting the angle of this part of the helix to be smaller than 5 degrees, the flow velocity of the sterilizer becomes irregular, and the vaporization efficiency is improved.

The helical metal pipe 2 is formed from a metal pipe worked into a helical shape that has an inner diameter equal to or greater than 3 mm and equal to or smaller than 30 mm and a thickness equal to or greater than 0.5 mm and equal to or smaller than 5 mm. The radius of the helical metal pipe 2 from the axial center thereof to the outermost part of the helical metal pipe 2 is appropriately equal to or greater than 10 mm and equal to or smaller than 200 mm. The dimension of the helical shape in the axial direction is appropriately equal to or greater than 50 mm and equal to or smaller than 800 mm. The total length of the helical metal pipe 2 is appropriately equal to or greater than 500 mm and equal to or smaller than 5000 mm. The ratio of the radius of the helix described above to the outer diameter of the helical metal pipe 2, that is, the radius of the helix/the outer diameter of the helical metal pipe 2, is preferably equal to or greater than 4 and equal to or smaller than 50. More preferably, the ratio is equal to or greater than 10 and equal to or smaller than 30. In this range, the ease of working into the helical shape and the size of the helical metal pipe 2 are well balanced. The nozzle 5 is connected to one end of the helical metal pipe 2. The other end of the helical metal pipe 2 serves as the sterilizer gas ejection port 6 through which the resulting sterilizer gas is ejected.

As shown in FIG. 4, the direction of the helix of the helical metal pipe 2 may change once. If the direction of the helix is changed, the flow of the sterilizer in the helical metal pipe 2 changes, and small droplets of the sterilizer that have not been gasified yet can come into contact with the inner surface of the helical metal pipe 2, so that the gasification of the sterilizer is promoted.

The helical metal pipe 2 is embedded in a molten metal. As shown in FIG. 1, the helical metal pipe 2 is embedded in the solidified molten metal layer 3 that covers the outer periphery of the helical pipe. In such a manner that the outer wall of the solidified molten metal layer 3 is formed at a distance equal to or greater than 5 mm and equal to or smaller than 30 mm from the outermost periphery of the helical pipe, the inside of the helical metal pipe 2 having the shape of a circular column is totally filled with a molten metal, and the helical part of the helical metal pipe 2 is embedded in the molten metal, while the two end parts of the helical metal pipe 2 are not embedded in the molten metal. The solidified molten metal layer 3 is formed by solidification of the molten metal.

The volume ratio between the helical metal pipe 2 and the solidified molten metal layer 3 has an effect on the vaporization efficiency. The volume ratio of the metal pipe 2 to the solidified molten metal layer 3 is preferably 1:3 to 1:30, and more preferably 1:5 to 1:15. If the volume ratio is lower than 1:3, the heat transfer effect from the heating body 4 decreases, and the amount of the sterilizer gas produced decreases. On the other hand, if the volume ratio is higher than 1:30, the proportion of the solidified molten metal layer is too high, and it is difficult to reduce the size and weight of the apparatus.

The solidified molten metal layer 3 is appropriately made of a metal having a relatively low melting point, such as brass, aluminum, zinc, magnesium, or an aluminum alloy. In particular, from the viewpoint of strength and thermal conductivity, brass, aluminum and aluminum alloys are appropriate. The melting point of aluminum is about 660°C, and the aluminum alloy has a lower melting point. Aluminum or an aluminum alloy is molten, poured into a mold in which the helical metal pipe 2 is held, and cooled and solidified to form the solidified molten metal layer 3.

The process of melting aluminum or an aluminum alloy and pouring the molten aluminum or aluminum alloy into a mold may be a casting process or die-casting process. The casting processes include the sand mold casting process, the metal mold casting, and the low pressure casting process. The die-casting processes include the normal die-casting process, the squeeze casting process, and the vacuum die-casting process. Although any of these processes can be used, the sand mold casting process, which is inexpensive and does not require strict dimensional accuracy, can be used.

The nozzle 5 that sprays the sterilizer into the helical metal pipe 2 is provided at one end of the helical metal pipe 2. The nozzle 5 can be any nozzle that can sprays the sterilizer into the helical metal pipe 2. However, the nozzle 5 is preferably a two-fluid spray. When the nozzle 5 is a two-fluid spray, the nozzle 5 is supplied with the sterilizer through a sterilizer supply port and with compressed air through a compressed air supply port. The nozzle 5 sprays a mist of the sterilizer to the inner surface of the helical metal pipe 2. The sprayed sterilizer is gasified by coming into contact with the inner surface of the helical metal pipe 2 that is heated by the heat transferred from the heating body 4 through the solidified molten metal layer 3. The gasified sterilizer is ejected from the sterilizer gas ejection port 6 in the form of a gas. The ejection is caused by the pressure of the compressed air supplied to the nozzle 5 and the volume expansion of the gasified sterilizer.

Although the nozzle 5 and the sterilizer gas ejection port 6 are provided at the center of the helical part of the helical metal pipe 2 in FIG. 1, the nozzle 5 and the sterilizer gas ejection port 6 may be provided at a position along the helical shape.

The sterilizer gas ejected from the sterilizer gas ejection port 6 can be directly blown on a container passing below the sterilizer gas ejection port 6. However, as shown in FIG. 1, a gasification apparatus tip end nozzle 10 that guides the ejected sterilizer gas may be provided at the tip end of the sterilizer gas ejection port 6, and the sterilizer gas may be blown from the tip end of the gasification apparatus tip end nozzle 10 on the container passing below the gasification apparatus tip end nozzle 10.

As with the helical metal pipe 2, the gasification apparatus tip end nozzle 10 is made of a metal, such as iron, stainless steel, copper, brass, or titanium, and can also be made of aluminum. Stainless steel, which has high durability against sterilizers and cleaning liquids containing chemicals, is preferable. The stainless steel may be an alloy of iron, chromium and nickel, an alloy of iron, chromium, nickel and molybdenum, an alloy of iron, chromium, nickel, molybdenum and copper, an alloy of iron, chromium, nickel, molybdenum and nitrogen, or a duplex stainless steel alloy having two structures, that is, any of the stainless steel alloys described above and ferrite.

The diameter of the tip end of the gasification apparatus tip end nozzle 10 is preferably approximately equal to or up to 30% smaller than the diameter of the sterilizer gas ejection port 6 of the helical metal pipe 2. If the diameter of the tip end of the gasification apparatus tip end nozzle 10 is more than 30% smaller than the diameter of the sterilizer gas ejection port 6, the blow range of the sterilizer gas is too narrow, and there is a possibility that the sterilization is insufficient. The distance between the tip end of the gasification apparatus tip end nozzle 10 and the container to be sterilized is equal to or smaller than 20 mm, and preferably equal to or smaller than 10 mm.

If the temperature of the gasification apparatus tip end nozzle 10 decreases, the sterilizer gas may be condensed at the part, and the condensate may drop. To prevent this, the gasification apparatus tip end nozzle 10 may be brought into contact with at least one of the solidified molten metal layer 3 or the heating body 4 for heating. Alternatively, a heat generating body may be brought into contact with the gasification apparatus tip end nozzle 10. When the heat generating body is brought into contact with the gasification apparatus tip end nozzle 10, the gasification apparatus tip end nozzle 10 may be provided with a sealing material, such as a gasket or an O-ring, in order to prevent leakage of the sterilizer gas. The material of the sealing material is preferably fluorocarbon rubber.

As operating conditions of the nozzle 5, for example, the pressure of the compressed air is adjusted in a range between 0.05 MPa and 0.8 MPa inclusive, the air flowrate is appropriately equal to or higher than 10 L/min and equal to or lower than 500 L/min, and preferably equal to or higher than 30L/min and equal to or lower than 300 L/min. If the air flowrate is lower than 10 L/min, it is difficult for the sterilizer to reach the bottom of the container when the sterilizer is blown into the container passing below the sterilizer gas ejection port 6 through the mouth portion of the container. In addition, the sterilizer is unevenly distributed in the container, and the sterilization effect varies. To avoid this, the gasification apparatus tip end nozzle 10 provided at the tip of the sterilizer gas ejection port 6 may be inserted into the container, although this leads to a more complicated apparatus and an increased cost. On the other hand, if the air flowrate is equal to or higher than 500 L/min, the concentration of the sterilizer decreases, and the sterilizer needs to be excessively supplied in order to compensate for the decrease of the concentration, so that the efficiency decreases. The sterilizer may be supplied by gravitation, under pressure from a pump, or under pressure of compressed air. The amount of supply of the sterilizer can be arbitrarily set, and the sterilizer is supplied in a range between 1 g/min and 200 g/min inclusive, for example.

The flowrate of the sterilizer gas can be appropriately adjusted according to the conveyance speed of the container. Specifically, when the total length of the container is equal to or greater than 100 mm and equal to or smaller than 400 mm, the inner diameter of the mouth of the container is equal to or greater than 15 mm and equal to or smaller than 40 mm, and the conveyance speed of the container is equal to or higher than 500 mm/sec and equal to or lower than 800 mm/sec, the flow velocity of the sterilizer gas at the tip of the sterilizer gas ejection port 6 or the gasification apparatus tip end nozzle 10 is appropriately equal to or higher than 10 m/sec and equal to or lower than 100 m/sec. When the total length of the container is equal to or greater than 30 mm and equal to or smaller than 300 mm, the inner diameter of the mouth of the container is equal to or greater than 15 mm and equal to or smaller than 40 mm, and the conveyance speed of the container is equal to or higher than 800 mm/sec and equal to or lower than 2500 mm/sec, the flow velocity of the sterilizer gas is appropriately equal to or higher than 30 m/sec and equal to or lower than 600 m/sec. If the flow velocity of the sterilizer gas is below this range, the sterilizer cannot adequately reach the bottom of the container, and the sterilization effect decreases. If the flow velocity is above this range, the gasification efficiency of the sterilizer decreases, and the gasification apparatus needs to be increased in size, which leads to an increase in cost.

The sterilizer preferably contains at least hydrogen peroxide. The content of hydrogen peroxide is appropriately in the range between 0.5% by mass and 650 by mass inclusive. If the content is lower than 0.5% by mass, the sterilizing power may be insufficient. If the content is higher than 65% by mass, the sterilizer is difficult to treat from the viewpoint of safety. The content is more preferably equal to or higher than 0.50 by mass and equal to or lower than 40% by mass. If the content is equal to or lower than 40% by mass, the sterilizer is easier to treat, and the amount of the residual hydrogen peroxide on the packaging material after sterilization can be reduced because of the low concentration.

The sterilizer contains a stabilizer in order to prevent decomposition of hydrogen peroxide. The stabilizer contained in the sterilizer is preferably sodium pyrophosphate or orthophosphoric acid, which are designated food additives for sterilizing packaging materials for foods designated by the Minister of Health, Labour and Welfare. However, a phosphorus-containing inorganic compound such as sodium hydrogen pyrophosphate or a phosphonic chelating agent such as aminotrimethylene phosphonic acid or alkylidene di-phosphonate can also be used. The content of the stabilizer is typically equal to or lower than 40 ppm and may be as low as 10 ppm or lower.

The sterilizer contains water. However, the sterilizer may contain one or more of alcohols such as methyl alcohol, ethyl alcohol, isopropyl alcohol, n-propyl alcohol and butyl alcohol, ketones such as acetone, methyl ethyl ketone and acetylacetone, and glycol ether and the like.

The sterilizer may further contain a compound having a sterilization effect such as peracetic acid, acetic acid, chlorine compound or ozone, or an additive such as a cationic surface active agent or a non-ionic surface active agent.

The nozzle 5 may be directly connected to the helical metal pipe 2, or an extension pipe may be provided between the helical metal pipe 2 and the nozzle 5. The extension pipe is provided to prevent the heat of the helical metal pipe 2 from being transferred to the nozzle 5 to raise the temperature of the nozzle 5.

The sterilizer gas ejected from the sterilizer gas ejection port 6 may be directly blown on the container or other packaging material to be sterilized. However, the sterilizer gasified in the helical metal pipe 2 and ejected from the sterilizer gas ejection port 6 may be mixed in a conduit with heated air, which is air from a blower heated by a heating apparatus, and the sterilizer gas mixed with the heated air may be blown on the container or other packaging material to be sterilized. Not only a single sterilizer gasification apparatus but also a plurality of sterilizer gasification apparatuses may be coupled to the conduit. The heating apparatus heats air to a temperature equal to or higher than 130°C and equal to or lower than 300°C.

The sterilizer gas ejection port 6 may be provided with a filter that captures a deposited stabilizer. Although the stabilizer used has no hygienic problem, the deposited stabilizer may adhere to the sterilizer gas ejection port 6 and cause a blockage of the sterilizer gas ejection port 6. The filter can be any filter that has a heat resistance up to 300°C and can capture the deposited stabilizer, such as a ceramic filter made of alumina, zirconium oxide, titanium oxide or the like having an average pore diameter equal to or greater than 0.1 µm and equal to or smaller than 20 µm, or a nonwoven fabric made of an inorganic substance, cellulose or the like.

The sterilizer gas ejection port 6 may be provided with a branch pipe, on which a hydrogen peroxide gas concentration meter, a thermometer or a conductivity meter may be installed. By providing the hydrogen peroxide gas concentration meter and the thermometer, the concentration of the hydrogen peroxide gas and the temperature of the sterilizer gas, which are essential for sterilization, can be constantly or irregularly measured. In general, the hydrogen peroxide gas concentration meter has low heat resistance, so that it is preferable to measure the gas temperature and the hydrogen peroxide gas concentration after decreasing the gas temperature to less than 100°C and decreasing the hydrogen peroxide gas concentration to 20 mg/L (preferably 10 mg/L). The conductivity meter has an advantage that after the interior of the sterilizer gasification apparatus 1 is cleaned with a cleaning liquid containing a chemical, it can be checked in the water rinsing step that there is no residue of the chemical.

The heating body 4 that heats the outer surface of the solidified molten metal layer 3 is provided on the outer side of the solidified molten metal layer 3. The heating body 4 is a cast heater worked so that the heater can come into contact with the outer surface of the solidified molten metal layer 3. Any process can be used, such as using a plate-like heater, flowing a current to the heating body 4 to make the heating body 4 itself generate heat, or using an induction heating apparatus for heating, as far as the solidified molten metal layer 3 can be heated to a desired temperature. The heating body 4 is heated to a temperature equal to or higher than 130°C and equal to or lower than 500°C. An outer armor for heat insulation may be provided on the outer side of the heating body 4.

When a cast heater is used, the distance between the cast in place heater and the inner periphery of the heating body 4 is preferably shorter than the distance between the cast in place heater and the outer periphery of the heating body 4, in order to improve the thermal conductivity. In other words, the cast heater can be located toward the inner side of the heating body 4.

The heating body 4 may be divided into two or more parts between the upstream side and the downstream side of the sterilizer gasification apparatus 1. This is intended to divide the weight of the heating body 4 to reduce the burden on the workers. In addition, the temperature of each part can be independently set, so that an appropriate temperature can be set for each part of the sterilizer gasification apparatus 1. For example, the temperature of a part near the sterilizer inlet may be set low so that the temperature of the sterilizer can be smoothly raised. And the temperature of a part near the sterilizer outlet may be set high so that the temperature of the sterilizer is further raised so that the sterilizer is less likely to be condensed.

The heating body 4 is appropriately made of a metal having a relatively low melting point, such as brass, aluminum, zinc, magnesium, or an aluminum alloy. In particular, from the viewpoint of strength and thermal conductivity, brass, aluminum and aluminum alloys are appropriate. The melting point of aluminum is about 660°C, and the aluminum alloy has a lower melting point. Aluminum or an aluminum alloy is molten, poured into a mold in which heater wire is held, and cooled and solidified to form the heating body 4.

When the heating body 4 is heated to 130°C or higher, adiabatic expansion of aluminum or other metal occurs. Therefore, the heating body 4 and the solidified molten metal layer 3 are preferably made of the same material, so that the gap formed between the heating body 4 and the solidified molten metal layer 3 while heating can be reduced.

By heating the heating body 4 to a temperature equal to or higher than 130°C and equal to or lower than 500°C, the inner surface of the helical metal pipe 2 can be maintained at a temperature equal to or higher than 130°C and equal to or lower than 500°C while the sterilizer is being sprayed. When the sterilizer contains 35% by mass of hydrogen peroxide, the concentration of hydrogen peroxide in the sterilizer gas is equal to or higher than 200 mg/L and equal to or lower than 500 mg/L.

Since the helical metal pipe 2 is heated by the heating body 4, the sterilizer gas ejection port 6 can be heated with reliability. Therefore, compared with the conventional gasification apparatus having a cylindrical shape, the sterilizer gas is prevented from liquefying near the sterilizer gas ejection port 6, and the sterilizer gasified in the helical metal pipe 2 can be kept gasified and blown on the object to be sterilized.

As the helical metal pipe 2 of the sterilizer gasification apparatus 1 is used for a long time, the stabilizer contained in the hydrogen peroxide solution may be deposited and accumulated in the helical metal pipe 2 or the sterilizer gas ejection port 6. The accumulated stabilizer inhibits the heat transfer to the internal surface of the helical metal pipe 2, and decreases the gasification efficiency of the sterilizer. In addition, if the stabilizer is excessively accumulated, the accumulated stabilizer may corrode the stainless steel pipe or cause a blockage of the helical metal pipe 2 or the sterilizer gas ejection port 6. The part including the helical metal pipe 2 and the solidified molten metal layer 3 according to this embodiment is relatively inexpensive, so that if the part is replaced after a certain period of use, and the nozzle 5 and the heating body 4 are reused, the sterilizer gasification apparatus 1 can be operated at low cost. Furthermore, unlike the conventional gasification apparatus, the sterilizer gasification apparatus 1 does not require cleaning, and the aseptic filling machine can be restarted in a short time.

To elongate the replacement interval of the part including the helical metal pipe 2 and the solidified molten metal layer 3, the interior of the helical metal pipe 2 is preferably cleaned.

The sterilizer gasification apparatus 1 according to this embodiment has the helical metal pipe 2 as a part that vaporizes the sterilizer and therefore is superior in cleaning effect with a cleaning liquid and in ease of rinsing, compared with the conventional gasification apparatus having a cylindrical shape. That is, with the sterilizer gasification apparatus 1, the deposited stabilizer can be efficiently removed in a short time, and the chemical used in the cleaning liquid can be efficiently rinsed away in a short time. After the operation of the gasification apparatus 1 ends, the stabilizer is removed by supplying a cleaning liquid containing an alkaline or acidic chemical to the nozzle 5 that sprays the sterilizer and spraying the cleaning liquid into the helical metal pipe 2. Water is then supplied to rinse the chemical away. Water may be supplied before the cleaning liquid containing an alkaline or acidic chemical is supplied.

As the cleaning liquid containing an alkaline chemical, water containing 0.5% by mass to 5% by mass of sodium hydroxide and 0.1% by mass to 5% by mass of chelating agent as additives is preferably used. The interior of the sterilizer gasification apparatus 1 immediately after the operation of the sterilizer gasification apparatus 1 ends is at a high temperature equal to or higher than 50°C and equal to or lower than 300°C, and thus, as the water, ion-exchanged water, reverse osmosis-filtered water or distilled water is preferably used since deposition of calcium, magnesium and the like does not occur. After rinsing with water, air is supplied from the nozzle 5 or a cleaning liquid pouring apparatus 7 to blow air into the sterilizer gasification apparatus 1 to remove any remaining water. The remaining water blown by air is discharged into the aseptic filling machine, so that the air to be supplied is preferably aseptic air having passed through a filter.

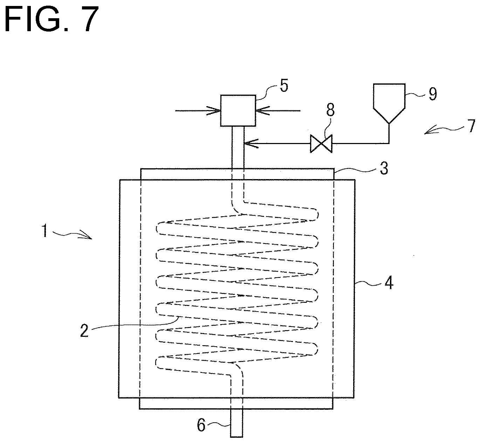

As shown in FIG. 7, the cleaning liquid pouring apparatus 7 that pours the cleaning liquid into the helical metal pipe 2 may be provided downstream of the nozzle 5. The cleaning liquid pouring apparatus 7 pours the cleaning liquid into the helical metal pipe 2 from a cleaning liquid storage tank 9 by opening and closing a cleaning liquid pouring valve 8. The cleaning liquid is a solution containing an alkaline or acidic chemical or water. As the water, ion-exchanged water, reverse osmosis-filtered water or distilled water is preferably used since deposition of calcium, magnesium and the like does not occur. The helical metal pipe 2 is preferably made of a material that has a resistance to the chemicals described above, such as stainless steel or titanium. The stainless steel may be an alloy of iron, chromium and nickel, an alloy of iron, chromium, nickel and molybdenum, an alloy of iron, chromium, nickel, molybdenum and copper, an alloy of iron, chromium, nickel, molybdenum and nitrogen, or a duplex stainless steel alloy having two structures, that is, any of the stainless steel alloys described above and ferrite. Specifically, the stainless steel may be SUS316L, SUS821L, SUS329J1, SUS323L, SUS329J3L, SUS329J4L or SUS327L1.

The cleaning liquid stored in the cleaning liquid storage tank 9 is poured into the helical metal pipe 2 by gravitation, under pressure from a pump or under pressure of compressed air, for example. After the spraying of the sterilizer ends, the cleaning liquid pouring valve 8 is opened to pour the cleaning liquid into the helical metal pipe 2. A nozzle may be provided at the tip end of the cleaning liquid pouring apparatus 7, and the cleaning liquid may be sprayed into the helical metal pipe 2.

The gasification apparatus according to the embodiment 1 of the present disclosure is lighter than the conventional gasification apparatus. In addition, for the same temperature of the surface that comes into contact with the sterilizer and the same amount of hydrogen peroxide solution supplied, the gasification apparatus according to the embodiment 1 produces a larger amount of hydrogen peroxide and has higher gasification efficiency than the conventional gasification apparatus.

### (Embodiment 2)

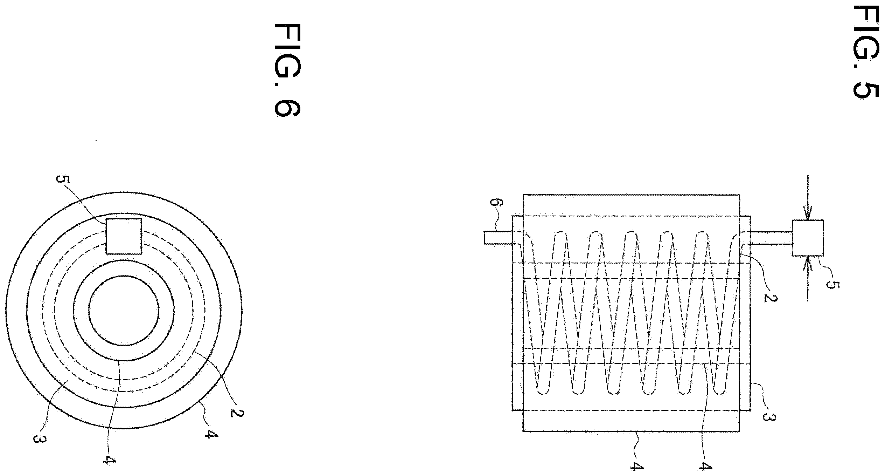

FIG. 5 shows a sterilizer gasification apparatus 1 according to an embodiment 2. The helical metal pipe 2 is covered on the outer periphery side and the inner periphery side, and the solidified molten metal layer 3 has the shape of a cylindrical tube. While the solidified molten metal layer 3 in the embodiment 1 has the shape of a cylindrical column, the solidified molten metal layer 3 in the embodiment 2 has the shape of a cylindrical tube, and heating bodies 4 are provided that heat the outer and inner surfaces of the solidified molten metal layer 3 having the shape of a cylindrical tube. In this case, the radius of the helix of the helical metal pipe 2 is appropriately equal to or greater than 50 mm and equal to or smaller than 300 mm with respect to the central axis of the helical shape of the helical metal pipe 2. That is, the diameter of the helix is increased so that a space can be provided inside the helical metal pipe 2. By providing heating bodies 4 not only on the outer side but also on the inner side of the cylindrical tube, the helical metal pipe 2 can be efficiently heated. By increasing the quantity of heat for heating the helical metal pipe 2, the amount of the sterilizer supplied to the metal pipe 2 can be increased, and the amount of the sterilizer gas for sterilizing the container can be increased.

FIG. 6 is a plan view of the sterilizer gasification apparatus 1 according to the embodiment 2. The helical metal pipe 2 is covered by the solidified molten metal layer 3 on the outer and inner sides thereof, and the heating bodies 4 are provided on the outer and inner peripheries of the solidified molten metal layer 3. By providing the heating bodies 4 on the outer and inner peripheries of the solidified molten metal layer 3, the inner and outer surfaces of the helical metal pipe 2 can be heated.

Embodiments of the present disclosure are configured as described above. However, the present disclosure is not limited to the embodiments described above, and various modifications can be made without departing from the spirit of the present disclosure.

### Reference Signs List

- 1: sterilizer gasification apparatus
- 2: helical metal pipe
- 3: solidified molten metal layer
- 4: heating body
- 5: nozzle
- 6: sterilizer gas ejection port
- 7: cleaning liquid pouring apparatus
- 10: gasification apparatus tip end nozzle

## Claims

1. A sterilizer gasification apparatus, comprising:
a helical metal pipe that meets at least one of conditions that a pitch of a helical pipe of the helical metal pipe is equal to or greater than 1.0 mm and equal to or smaller than 30.0 mm and that an angle of a helix of the helical metal pipe is greater than 0 degrees and equal to or smaller than 30 degrees with respect to a plane perpendicular to a central axis of the helix;
a solidified molten metal layer in which a helical part of the helical metal pipe is embedded in a casting process, two end parts of the helical metal pipe not being embedded in the solidified molten metal layer;
a heating body that heats an outer surface of the solidified molten metal layer; and
a nozzle that is provided at one end of the helical metal pipe and sprays a sterilizer into the helical metal pipe.

2. The sterilizer gasification apparatus according to Claim 1, wherein the helical metal pipe is made of stainless steel.

3. The sterilizer gasification apparatus according to Claim 1, wherein a sterilizer gas ejection port, from which a sterilizer gas is ejected, at another end of the helical metal pipe is provided with a gasification apparatus tip end nozzle that guides the ejected sterilizer gas.

4. The sterilizer gasification apparatus according to Claim 3, wherein the gasification apparatus tip end nozzle is made of stainless steel or aluminum.

5. The sterilizer gasification apparatus according to any one of Claims 1 to 4, wherein a direction of the helix of the helical metal pipe changes at least once.

6. The sterilizer gasification apparatus according to any one of Claims 1 to 4, wherein the solidified molten metal layer is made of brass, aluminum or an aluminum alloy.

7. The sterilizer gasification apparatus according to any one of Claims 1 to 4, wherein the solidified molten metal layer and the heating body that heats the outer surface of the solidified molten metal layer are made of a same material.

8. The sterilizer gasification apparatus according to any one of Claims 1 to 4, wherein the solidified molten metal layer has the shape of a circular column and covers an outer periphery and an inside of the helical metal pipe.

9. The sterilizer gasification apparatus according to any one of Claims 1 to 4, wherein the solidified molten metal layer has the shape of a circular tube and covers an outer periphery and an inner periphery of the helical metal pipe.

10. The sterilizer gasification apparatus according to any one of Claims 1 to 4, wherein the nozzle is a two-fluid spray.

11. The sterilizer gasification apparatus according to any one of Claims 1 to 4, wherein a cleaning liquid pouring apparatus that pours a cleaning liquid into the helical metal pipe is provided.

12. The sterilizer gasification apparatus according to any one of Claims 1 to 4, wherein a sterilizer gas ejection port from which a gas of the sterilizer gasified in the helical metal pipe is ejected is provided with a branch pipe, on which one or more of a hydrogen peroxide gas concentration meter, a thermometer and a conductivity meter is provided.

13. A sterilizer gasification method, wherein a helical metal pipe that meets at least one of conditions that a pitch of a helical pipe of the helical metal pipe is equal to or greater than 1.0 mm and equal to or smaller than 30.0 mm and that an angle of a helix of the helical metal pipe is greater than 0 degrees and equal to or smaller than 30 degrees with respect to a plane perpendicular to a central axis of the helix, a solidified molten metal layer in which a helical part of the helical metal pipe is embedded in a casting process, two end parts of the helical metal pipe not being embedded in the solidified molten metal layer, and a heating body that heats an outer surface of the solidified molten metal layer are provided, and
a sterilizer is sprayed into the helical metal pipe from one end of the helical metal pipe.

14. The sterilizer gasification method according to Claim 13, wherein the helical metal pipe is made of stainless steel.

15. The sterilizer gasification method according to Claim 13, wherein a sterilizer gas ejection port, from which a sterilizer gas is ejected, at another end of the helical metal pipe is provided with a gasification apparatus tip end nozzle to guide the ejected sterilizer gas.

16. The sterilizer gasification method according to Claim 15, wherein the gasification apparatus tip end nozzle is made of stainless steel or aluminum.

17. The sterilizer gasification method according to any one of Claims 13 to 16, wherein a direction of the helix of the helical metal pipe changes at least once.

18. The sterilizer gasification method according to any one of Claims 13 to 16, wherein the solidified molten metal layer is made of brass, aluminum or an aluminum alloy.

19. The sterilizer gasification method according to any one of Claims 13 to 16, wherein the solidified molten metal layer and the heating body that heats the outer surface of the solidified molten metal layer are made of a same material.

20. The sterilizer gasification method according to any one of Claims 13 to 16, wherein the solidified molten metal layer has the shape of a circular column and covers an outer periphery and an inside of the helical metal pipe.

21. The sterilizer gasification method according to any one of Claims 13 to 16, wherein the solidified molten metal layer has the shape of a circular tube and covers an outer periphery and an inner periphery of the helical metal pipe.

22. The sterilizer gasification method according to any one of Claims 13 to 16, wherein the sterilizer is sprayed into the helical metal pipe by a two-fluid spray.

23. The sterilizer gasification method according to any one of Claims 13 to 16, wherein a cleaning liquid is poured into the helical metal pipe.

24. The sterilizer gasification method according to Claim 23, wherein water used for the cleaning liquid is ion-exchanged water, reverse osmosis-filtered water or distilled water.

25. The sterilizer gasification method according to Claim 23, wherein after the cleaning liquid is poured into the helical metal pipe, the cleaning liquid is rinsed away with water, and air is blown into the helical metal pipe to remove remaining water.
